# EUROPÄISCHE PATENTSCHRIFT

(11) **EP 1 530 462 B1**
(45) Veröffentlichungstag und Bekanntmachung des Hinweises auf die Patenterteilung: **15.12.2010**
(21) Anmeldenummer: 03765006.6
(22) Anmeldetag: 16.07.2003
(51) Int. Cl.: A61K 31/135, C07C 215/46, A61P 25/04, A61P 25/24

(54) **1-PHENYL-2-DIMETHYLAMINOMETHYLCYCLOHEXANVERBINDUNGEN ZUR THERAPIE VON DEPRESSIVEN SYMPTOMATIKEN, SCHMERZ UND INKONTINENZ**
1-PHENYL-2-DIMETHYLAMINOMETHYL CYCLOHEXANE COMPOUNDS USED FOR THE THERAPY OF DEPRESSIVE SYMPTOMS, PAIN, AND INCONTINENCE
COMPOSES DE 1-PHENYL-2-DIMETHYLAMINOMETHYLCYCLOHEXANE DESTINES AU TRAITEMENT DES SYMPTOMES DEPRESSIFS, DES DOULEURS ET DE L'INCONTINENCE

(30) Priorität: 19.07.2002 DE 10233048
(43) Veröffentlichungstag der Anmeldung: 18.05.2005
(73) Patentinhaber: Grünenthal GmbH, 52078 Aachen (DE)
(72) Erfinder: FRIDERICHS, Elmar, 52223 Stolberg (DE); STRASSBURGER, Wolfgang, 52146 Würselen (DE); JAHNEL, Ulrich, 52379 Langerwehe (DE); ZIMMER, Dr. Oswald, 52146 Würselen (DE); SAUNDERS, Dr. Derek, 52072 Aachen (DE); ENGLBERGER, Dr. Werner, 52223 Stolberg (DE); HENNIES, Dr. Hagen-Heinrich, 52152 Simmerath (DE); BUSCHMANN, Dr. Helmut, 08950 Esplugues de Llobregat (ES); HOLENZ, Jörg, 008800 Vilanova i la geltru (ES)
(74) Vertreter: Brosch, Oliver
(86) Internationale Anmeldenummer: PCT/EP2003/007720
(87) Internationale Veröffentlichungsnummer: WO 2004/009067

(56) Entgegenhaltungen:
- DE-A- 10 059 411
- DE-A- 19 525 137
- ROJAS-CORRALES M O ET AL: "TRAMADOL INDUCES ANTIDEPRESSANT-TYPE EFFECTS IN MICE" LIFE SCIENCES, PERGAMON PRESS, OXFORD, GB, Bd. 63, Nr. 12, 1998, Seiten 175-180, XP000910402 ISSN: 0024-3205
- SPENCER C: "THE EFFICACY OF INTRAMUSCULAR TRAMADOL AS A RAPID-ONSET ANTIDEPRESSANT" AUSTRALIAN AND NEW ZEALAND JOURNAL OF PSYCHIATRY, MELBOURNE, AU, Bd. 34, Nr. 6, Dezember 2000 (2000-12), Seite 1032, XP001097637 ISSN: 0004-8674
- RUDAZ S ET AL: "Simultaneous stereoselective analysis by capillary electrophoresis of tramadol enantiomers and their main phase I metabolites in urine" JOURNAL OF CHROMATOGRAPHY A, ELSEVIER SCIENCE, NL, Bd. 846, Nr. 1-2, 18. Juni 1999 (1999-06-18), Seiten 227-237, XP004170405 ISSN: 0021-9673
- FLICK K ET AL: "[Studies on chemical structure and analgetic activity of phenyl substituted aminomethylcyclohexanoles (author's transl)]" ARZNEIMITTEL-FORSCHUNG. 1978, Bd. 28, Nr. 1a, 1978, Seiten 107-113, XP0000608150 ISSN: 0004-4172

## Beschreibung

Die Erfindung betrifft Metabolite von [2-(3-Methoxyphenyl)-cyclohexylmethyl]-dimethylamin als freie Basen und/oder in Form physiologisch verträglicher Salze, entsprechende Arzneimittel sowie die Verwendung von [2-(3-Methoxyphenyl)-cyclohexylmethyl]-dimethylamin und seiner Metabolite zur Herstellung eines Arzneimittels zur Behandlung von Depressionen.

Depression ist eine Störung der Affektivität, bei der ein depressives Syndrom im Vordergrund steht, wobei depressiv mit Verstimmung verbunden bzw. traurig gestimmt heißt. Die zur Therapie verwendeten Antidepressiva sind auch wichtige Adjuvantien für die Schmerztherapie (R. van Schayck et al. 1998, MMP, 10, 304-313; Jung et al. 1997, J.Gen.Intern.Med.12, 384-389; Onghena and Van Houdenhove 1992, Pain, 49, 205-219; Feuerstein 1997, Diagnostik und Therapie, 3, 213-225; Rowbotham 1997, The Pain Medicine Journal Club Journal, 3, 119-122) insbesondere bei chronischen Schmerzzuständen, da die andauernde Schmerzbelastung zu einer depressiven Verstimmung der Patienten führen kann. Dies ist besonders häufig bei Krebs-Schmerzpatienten der Fall (Berard 1996, Int.Med.J., 3, 257-259). Da es bisher keine Schmerzmittel mit einer klinisch relevanten antidepressiven Wirkkomponenten gibt, müssen die Antidepressiva als Zusatzmedikation zur Analgetika-Gabe hinzugefügt werden. Da chronische Schmerzpatienten häufig eine Vielzahl von verschiedenen Medikamenten benötigen, führt die zusätzliche Gabe des Antidepressivums zu einer weiteren Belastung des Organismus. Aus diesem Grund und zur Verbesserung der Compliance wäre eine in einer analgetischen Substanz bereits vorhandene antidepressive Wirkkomponente von besonderem Vorteil.

Grundlage der antidepressiven Wirkung ist die Wiederaufnahmehemmung von Noradrenalin und Serotonin. Opioide mit einer mäßigen Uptakehemmung wie z. B. Tramadol sind seit längerem bekannt und werden als potente Analgetika mit einem geringen Sucht- und Abhängigkeitspotential therapeutisch eingesetzt (Raffa et al. 1992, Journal of Pharmacology and experimental Therapeutics, 260, 275-285; Raffa and Friderichs 1996, Pain Review, 3, 249-271). Die Uptakehemmkomponente ist jedoch nicht stark genug, um einen klinisch relevanten antidepressiven Effekt auslösen zu können.

Neben der eigentlichen antidepressiven Wirkung führt die Reuptakehemmung von Noradrenalin und Serotonin auch zu einer eigenständigen analgetischen Wirkung, in dem absteigende Schmerzhemmbahnen auf der Ebene des Rückenmarks aktiviert werden. In Kombination mit Opiaten, die ebenfalls auf der Rückenmarksebene die Schmerzweiterleitung hemmen, führt dies zu einer wechselseitige Potenzierung der Schmerzhemmmechanismen und damit zu einer besonders effektiven Analgesie.

Besonders günstig wäre daher in dieser Indikation, insbesondee der Behandlung von Depressionen gerade auch in Kombination mit Schmerztherapie, eine Opioidsubstanz mit einer relativ starken NA-und/oder 5HT-Uptake Hemmkomponente, die eine antidepressive Wirksamkeit zeigt (z.B. nachgewiesen in einem geeigneten tierexperimentellen Verhaltensmodell).

Aufgabe der vorliegenden Erfindung war es daher, Stoffe, insbesondere Opiodsubstanzen, aufzufinden, die zur Behandlung von Depressionen geeignet sind, insbesondere solche, die diese Wirksamkeit mit analgetischer Wirksamkeit kombinieren.

Überraschenderweise wurde nun gefunden, daß 2-(3-Methoxyphenyl)-cyclohexylmethyl)-dimethylamin wie auch seine Metabolite und dabei insbesondere das 3-(2-Dimethylaminomethyl-cyclohexyl)-phenol über eine therapeutisch relevante antidepressive Wirkkomponente verfügen.

Dementsprechend ist Erfindungsgegenstand die Verwendung von
■ 3-(2-Dimethylaminomethyl-cyclohexyl)-phenol,
■ (1R,2R)- 3-(2-Dimethylaminomethyl-cyclohexyl)-phenol,
■ [2-(3-Methoxyphenyl)-cyclohexylmethyl]-dimethylamin,
■ (1R, 2R)-[2-(3-Methoxyphenyl)-cyclohexylmethyl]-dimethylamin,
■ Schwefelsäure mono-[3-(2-dimethylaminomethyl-cyclohexyl)-phenyl]ester,
■ Schwefelsäure mono-(1R,2R)-[3-(2-dimethylaminomethyl-cyclohexyl)-phenyl]ester,
■ 3-(2-Methylaminomethyl-cyclohexyl)-phenol,
■ (1R,2R)-3-(2-Methylaminomethyl-cyclohexyl)-phenol,
■ 3-(2-Dimethylaminomethyl-cyclohexyl)-phenol-N-oxid,
■ (1R,2R)-3-(2-Dimethylaminomethyl-cyclohexyl)-phenol-N-oxid,
■ 6-[3-(2-Dimethylaminomethyl-cyclohexyl)-phenoxy]-3,4,5-trihydroxy-tetrahydropyran-2-carbonsäure,
■ 6-[(1R,2R)-3-(2-Dimethylaminomethyl-cyclohexyl)-phenoxy]-3,4,5-trihydroxy-tetrahydropyran-2-carbonsäure,
■ 4-(2-Dimethylaminomethyl-cyclohexyl)-catechol,
■ (1R,2R)-4-(2-Dimethylaminomethyl-cyclohexyl)-catechol,
■ 3-(2-Aminomethyl-cyclohexyl)-phenol,
■ (1R,2R)-3-(2-Aminomethyl-cyclohexyl)-phenol,
■ 4-(2-Dimethylaminomethyl-cyclohexyl)-benzene-1,2-diol,
■ (1R,2R)- 4-(2-Dimethylaminomethyl-cyclohexyl)-benzene-1,2-diol,
■ C-[2-(3-Methoxy-phenyl)-cyclohexyl]-methylamin,
■ (1R,2R)-C-[2-(3-Methoxy-phenyl)-cyclohexyl]-methylamin,
■ [2-(3-Methoxy-phenyl)-cyclohexylmethyl]-methyl-amin,
■ (1R,2R)-[2-(3-Methoxy-phenyl)-cyclohexylmethyl]-methyl-amin,
■ [2-(3-Methoxy-phenyl)-cyclohexylmethyl]-dimethyl-amin; N-oxid oder
■ (1R,2R)-[2-(3-Methoxy-phenyl)-cyclohexylmethyl]-dimethyl-amin; N-oxid;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
zur Herstellung eines Arzneimittels zur Behandlung von Depressionen.

Dabei ist es besonders bevorzugt, wenn die verwendeten Verbindungen als R, R, vorzugsweise 1R, 2R Stereoisomere vorliegen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Das bevorzugte Salz der verwendeten Verbindungen ist das Hydrochlorid.

Unter physiologisch verträglich ist zu verstehen, daß die Substanz, insbesondere das Salz als solches, bei Anwendung im Menschen oder Säugetier verträglich ist, also beispielsweise nicht unphysiologisch (z.B. giftig) wirkt.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, a-Liponsäure, Acetylglycin, Acetylsalicylsäure, Hippursäure und/oder Asparaginsäure. Besonders bevorzugt ist das Hydrochlorid-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, a-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali-und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch NH₄⁺, insbesondere aber (Mono-) oder (Di-) Natrium-, (Mono-) oder (Di-) Kalium-, Magnesium- oder Calzium-Salze.

Nach den vorliegenden Untersuchungen sind die verwendeten Substanzen und insbesondere (1R,2R)- 3-(2-Dimethylaminomethyl-cyclohexyl)-phenol potente Analgetika und Antidepressiva, verfügen also über eine zusätzliche und klinisch relevante antidepressive Wirkkomponente. Insbesondere (1R,2R)- 3-(2-Dimethylaminomethyl-cyclohexyl)-phenol kann bei mäßigen bis starken akuten und chronischen Schmerzen eingesetzt werden und ermöglicht die Behandlung der depressiven Begleitsymptomatik bei chronischen Schmerzzuständen. Sie führt damit zu einer deutlichen Verbesserung der chronischen Schmerzbehandlung, da bisher Antidepressiva als zusätzliche Medikamente zu denen der Schmerztherapie hinzugefügt werden mußten. (1R,2R)-3-(2-Dimethylaminomethyl-cyclohexyl)-phenol und die anderen erfindungsgemäß verwendeten Substanzen sind auch unabhängig von ihrer analgetischen Wirkung als genuine Antidepressiva anwendbar.

Ein weiterer Gegenstand der Erfindung sind Metabolite, insbesondere von [2-(3-Methoxyphenyl)-cyclohexylmethyl]-dimethylamin bzw. 3-(2-Dimethylaminomethyl-cyclohexyl)-phenol ausgewählt aus
■ Schwefelsäure mono-[3-(2-dimethylaminomethyl-cyclohexyl)-phenyl]ester,
■ Schwefelsäure mono-(1R,2R)-[3-(2-dimethylaminomethyl-cyclohexyl)-phenyl]ester,
■ 3-(2-Methylaminomethyl-cyclohexyl)-phenol,
■ (1R,2R)-3-(2-Methylaminomethyl-cyclohexyl)-phenol,
■ 3-(2-Dimethylaminomethyl-cyclohexyl)-phenol-N-oxid,
■ (1R,2R)-3-(2-Dimethylaminomethyl-cyclohexyl)-phenol-N-oxid,
■ 6-[3-(2-Dimethylaminomethyl-cyclohexyl)-phenoxy]-3,4,5-trihydroxy-tetrahydropyran-2-carbonsäure,
■ 6-[(1R,2R)-3-(2-Dimethylaminomethyl-cyclohexyl)-phenoxy]-3,4,5-trihydroxy-tetrahydropyran-2-carbonsäure,
■ 4-(2-Dimethylaminomethyl-cyclohexyl)-catechol,
■ (1R,2R)-4-(2-Dimethylaminomethyl-cyclohexyl)-catechol,
■ 3-(2-Aminomethyl-cyclohexyl)-phenol,
■ (1R,2R)-3-(2-Aminomethyl-cyclohexyl)-phenol,
■ 4-(2-Dimethylaminomethyl-cyclohexyl)-benzene-1,2-diol,
■ (1R,2R)- 4-(2-Dimethylaminomethyl-cyclohexyl)-benzene-1,2-diol,
■ C-[2-(3-Methoxy-phenyl)-cyclohexyl]-methylamin,
■ (1R,2R)-C-[2-(3-Methoxy-phenyl)-cyclohexyl]-methylamin,
■ [2-(3-Methoxy-phenyl)-cyclohexylmethyl]-methyl-amin,
■ (1R,2R)-[2-(3-Methoxy-phenyl)-cyclohexylmethyl]-methyl-amin,
■ [2-(3-Methoxy-phenyl)-cyclohexylmethyl]-dimethyl-amin; N-oxid oder
■ (1R,2R)-[2-(3-Methoxy-phenyl)-cyclohexylmethyl]-dimethyl-amin; N-oxid;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Besonders bevorzugt sind erfindungsgemäße Metabolite, die als R, R, vorzugsweise 1R, 2R Stereoisomere vorliegen.

Die erfindungsgemäßen Metabolite sind physiologisch unbedenklich. Daher ist ein weiterer Gegenstand der Erfindung ein Arzneimittel enthaltend als Wirkstoff wenigstens einen erfindungsgemäßen Metaboliten sowie gegebenenfalls Zusatz- und/oder Hilfsstoffe. Insbesondere ist entsprechend Gegenstand der Erfindung ein Arzneimittel enthaltend als Wirkstoff wenigstens eine Verbindung ausgewählt aus
■ Schwefelsäure mono-[3-(2-dimethylaminomethyl-cyclohexyl)-phenyl]ester,
■ Schwefelsäure mono-(1R,2R)-[3-(2-dimethylaminomethyl-cyclohexyl)-phenyl]ester,
■ 3-(2-Methylaminomethyl-cyclohexyl)-phenol,
■ (1R,2R)-3-(2-Methylaminomethyl-cyclohexyl)-phenol,
■ 3-(2-Dimethylaminomethyl-cyclohexyl)-phenol-N-oxid,
■ (1R,2R)-3-(2-Dimethylaminomethyl-cyclohexyl)-phenol-N-oxid,
■ 6-[3-(2-Dimethylaminomethyl-cyclohexyl)-phenoxy]-3,4,5-trihydroxy-tetrahydropyran-2-carbonsäure,
■ 6-[(1R,2R)-3-(2-Dimethylaminomethyl-cyclohexyl)-phenoxy]-3,4,5-trihydroxy-tetrahydropyran-2-carbonsäure,
■ 4-(2-Dimethylaminomethyl-cyclohexyl)-catechol,
■ (1R,2R)-4-(2-Dimethylaminomethyl-cyclohexyl)-catechol,
■ 3-(2-Aminomethyl-cyclohexyl)-phenol,
■ (1R,2R)-3-(2-Aminomethyl-cyclohexyl)-phenol,
■ 4-(2-Dimethylaminomethyl-cyclohexyl)-benzene-1,2-diol,
■ (1R,2R)- 4-(2-Dimethylaminomethyl-cyclohexyl)-benzene-1,2-diol,
■ C-[2-(3-Methoxy-phenyl)-cyclohexyl]-methylamin,
■ (1R,2R)-C-[2-(3-Methoxy-phenyl)-cyclohexyl]-methylamin,
■ [2-(3-Methoxy-phenyl)-cyclohexylmethyl]-methyl-amin,
■ (1R,2R)-[2-(3-Methoxy-phenyl)-cyclohexylmethyl]-methyl-amin,
■ [2-(3-Methoxy-phenyl)-cyclohexylmethyl]-dimethyl-amin; N-oxid oder
■ (1R,2R)-[2-(3-Methoxy-phenyl)-cyclohexylmethyl]-dimethyl-amin; N-oxid;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
sowie gegebenenfalls Zusatz- und/oder Hilfsstoffe.

Besonders bevorzugt ist es, wenn die enthaltenen Verbindungen als R, R, vorzugsweise 1R, 2R Stereoisomere vorliegen.

Geeignete Zusatz- und/oder Hilfsstoffe im Sinne dieser Erfindung sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäß verwendeten Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Weiter bevorzugt sind Arzneimittel, die wenigstens 0,05 bis 90,0 % des Wirkstoffes enthalten, insbesondere niedrige wirksame Dosierungen, um Neben- oder analgetische Wirkungen zu vermeiden. Üblicherweise werden 0,1 bis 5000 mg/kg, insbesondere 1 bis 500 mg/kg, vorzugsweise 2 bis 250 mg/kg Körpergewicht wenigstens einer erfindungsgemäß verwendeten Verbindung appliziert. Ebenso bevorzugt und üblich ist aber auch die Applikation von 0,01 - 5 mg/kg, vorzugsweise 0,03 bis 2 mg/kg, insbesondere 0,05 bis 1 mg/kg Körpergewicht.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur I oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Auch wenn die erfindungsgemässen Arzneimittel lediglich geringe Nebenwirkungen zeigen, kann es - so dies überhaupt notwendig sein sollte - beispielsweise zur Vermeidung von bestimmten Formen der Abhängigkeit von Vorteil sein, neben den verwendeten Verbindungen auch Morphinantagonisten, insbesondere Naloxon, Naltrexon und/oder Levallorphan, zu verwenden.

Da die erfindungsgemäßen Verbindungen - wie oben ausgeführt - analgetisch wirksam sind, ist ein weiterer Erfindungsgegenstand die Verwendung einer erfindungsgemäßen Verbindung, bzw. eines erfindungsgemäßen Metaboliten zur Herstellung eines Arzneimittels zur Behandlung von Schmerz, insbesondere akutem, viszeralem, chronischem oder neuropathischem Schmerz oder Krebsschmerz.

Da die erfindungsgemäßen Verbindungen darüberhinaus auch Wirksamkeit in der Harninkontinenz zeigen, ist ein weiterer Erfindungsgegenstand die Verwendung einer erfindungsgemäßen Verbindung, bzw. eines erfindungsgemäßen Metaboliten zur Herstellung eines Arzneimittels zur Behandlung von vermehrtem Harndrang bzw. Harninkontinenz.

Weiter offenbart die Erfindung auch ein Verfahren zur Behandlung von Depression, bei dem die erfindungsgemäß verwendeten Verbindungen verwendet werden.

Ebenso offenbart die Erfindung auch ein Verfahren zur Behandlung von Schmerz, insbesondere akutem, viszeralem, chronischem oder neuropathischem Schmerz oder Krebsschmerz, bei dem die erfindungsgemäßen Verbindungen, bzw. Metabolite verwendet werden.

Ebenso offenbart die Erfindung auch ein Verfahren zur Behandlung von vermehrtem Harndrang bzw. Harninkontinenz, bei dem die erfindungsgemäßen Verbindungen, bzw. Metabolite verwendet werden.

2-(3-Methoxyphenyl)-cyclohexylmethyl)-dimethylamin und (1R, 2R)-[2-(3-Methoxyphenyl)-cyclohexylmethyl)-dimethylamin] und ihre Herstellung sind aus der DE 195 25 137 A1 Beispiel 8 bzw. US 5,733,936 Example 8 bekannt, wobei die absolute Stereochemie der Verbindung (-6) hergestellt nach Beispiel 8 wohl korrekterweise (1R,2R) ist und nicht (1R,2S). Auch 3-(2-Dimethylaminomethyl-cyclohexyl)-phenol bzw. (1R,2R)-3-(2-Dimethylaminomethyl-cyclohexyl)-phenol und ihre Herstellung sind aus der DE 195 25 137 A1 Beispiel 10 bzw. US 5,733,936 Example 10 bekannt, wobei die absolute Stereochemie der Verbindung (-7) hergestellt nach Beispiel 10 wohl korrekterweise (1R,2R) ist und nicht (1R,2S).

Die Verbindungen, die und deren Herstellung noch nicht aus DE 195 25 137 A1 bzw. US 5,733,936 bekannt sind, wurden gemäß den Beispielen hergestellt.

Die folgenden Beispiele sollen die Erfindung erläutern, ohne daß der Gegenstand der Erfindung darauf beschränkt wäre.

### Beispiele

Generell wird Reinigung und Enantiomerentrennung bei allen als Beispiel genannten Verfahren auf verschiedenen Stufen mit Säulenchromatographie und überwiegend HPLC betrieben.

### Beispiel 1: Herstellung von (1R, 2R)-[2-(3-Methoxyphenyl)-cyclohexylmethyl]-methylamin, Hydrochlorid

[2-(3-Methoxyphenyl)-cyclohexylmethyl]-methylamin, bzw. (1R, 2R)-[2-(3-Methoxyphenyl)-cyclohexylmethyl]-methylamin, insbesondere deren Hydrochloridsalz, werden wie folgt hergestellt:

5,67 g (22,9 mmol) (1R, 2R)-[2-(3-Methoxyphenyl)-cyclohexylmethyl]-dimethylamin als freie Base wurden in 389,64 ml Toluol mit 3,16 ml (25,2 mmol) Phenylchloroformat 3h erhitzt. Nach Abkühlung und Waschen wurde der organische Rückstand eingeengt und mit 192,53 ml Ethylenglykol und insgesamt 45,84 ml 5N NaOH insgesamt 8,5h unter gelegentlichem Abkühlen bei 110°C gerührt. Es entstand: (1R, 2R)-[2-(3-Methoxyphenyl)-cyclohexylmethyl]-methylamin. Dieses wurde aufgearbeitet und mit TMCS als Hydrochlorid gefällt.

### Beispiel 2: Herstellung von (1R,2R)-3-(2-Methylaminomethyl-cyclohexyl)-phenol, Hydrochlorid

3,55 g (15,2 mmol) Hydrochloridsalz des (1R,2R)-[2-(3-Methoxy-phenyl)-cyclohexylmethyl]-methyl-amine gemäß Beispiel 1 wurden in 4,59 ml (47-48%) wässriger HBr 7,5 h unter Rückfluss gerührt und über Nacht abgekühlt. Es entsteht (1R,2R)-3-(2-Methylaminomethyl-cyclohexyl)-phenol. Dieses wurde aufgearbeitet und mit TMCS als Hydrochlorid gefällt.

### Beispiel 3: Herstellung von Schwefelsäure mono-(1R,2R)-[3-(2-dimethylaminomethyl-cyclohexyl)-phenyl]ester

Schwefelsäure mono-[3-(2-dimethylaminomethyl-cyclohexyl)-phenyl]ester bzw. Schwefelsäure mono-(1R,2R)-[3-(2-dimethylaminomethyl-cyclohexyl)-phenyl]ester wurde wie folgt hergestellt:

(1R,2R)- 3-(2-Dimethylaminomethyl-cyclohexyl)-phenol; Hydrochlorid wurde mit Dicyclohexylcarbodiimid (DCC) in H₂SO₄ behandelt. Es entstand Schwefelsäure mono-(1R,2R)-[3-(2-dimethylaminomethyl-cyclohexyl)-phenyl]ester.

### Beispiel 4: Herstellung von 6-[(1R,2R)-3-(2-Dimethylaminomethyl-cyclohexyl)-phenoxy]-3,4,5-trihydroxy-tetrahydropyran-2-carbonsäure

6-[3-(2-Dimethylaminomethyl-cyclohexyl)-phenoxy]-3,4,5-trihydroxy-tetrahydropyran-2-carbonsäure bzw. 6-[(1R,2R)-3-(2-Dimethylaminomethyl-cyclohexyl)-phenoxy]-3,4,5-trihydroxy-tetrahydropyran-2-carbonsäure wurde wie folgt hergestellt:

(1R,2R)- 3-(2-Dimethylaminomethyl-cyclohexyl)-phenol; Hydrochlorid wurde mit 3,4,5-Tri-O-acetyl-1-a-bromo-D-glucoronsäuremethylester mit 1. LiOH und 2. HOAc behandelt. Es entstand 6-[(1R,2R)-3-(2-Dimethylaminomethyl-cyclohexyl)-phenoxy]-3,4,5-trihydroxy-tetrahydropyran-2-carbonsäure. Die Aufreinigung erfolgte über Lobar-Lichoprep RP128 Säule MeOH:H2O-System und anschließender HPLC-Trennung.

### Beispiel 5: Herstellung von [2-(3-Methoxy-phenyl)-cyclohexylmethyl]-dimethyl-amin; N-oxid

[2-(3-Methoxy-phenyl)-cyclohexylmethyl]-dimethyl-amin; N-oxid bzw. (1R,2R)-[2-(3-Methoxy-phenyl)-cyclohexylmethyl]-dimethyl-amin; N-oxid wurde wie folgt hergestellt:

(1R,2R)-[2-(3-Methoxy-phenyl)-cyclohexylmethyl]-dimethyl-amin; Hydrochlorid wurde gelöst und bei Raumtemperatur mit H₂O₂ behandelt. Es entstand (1R,2R)-[2-(3-Methoxy-phenyl)-cyclohexylmethyl]-dimethyl-amin; N-oxid.

### Beispiel 6: Herstellung von (1R,2R)-3-(2-Dimethylaminomethyl-cychlohexyl)-phenol-N-oxid

3-(2-Dimethylaminomethyl-cychlohexyl)-phenol-N-oxid bzw. (1R,2R)-3-(2-Dimethylaminomethyl-cychlohexyl)-phenol-N-oxid wurde wie folgt hergestellt:

(1R,2R)-3-(2-Dimethylaminomethyl-cychlohexyl)-phenol; Hydrochlorid wurde gelöst und bei Raumtemperatur mit H₂O₂ behandelt. Es entstand (1R,2R)-3-(2-Dimethylaminomethyl-cychlohexyl)-phenol-N-oxid.

### Beispiel 7: Herstellung von 4-(2-Dimethylaminomethyl-cyclohexyl)-catechol oder 4-(2-Dimethylaminomethyl-cyclohexyl)-benzene-1,2-diol.

Die Herstellung von 4-(2-Dimethylaminomethyl-cyclohexyl)-catechol oder 4-(2-Dimethylaminomethyl-cyclohexyl)-benzene-1,2-diol erfolgte nach folgenden Reaktionsschema:

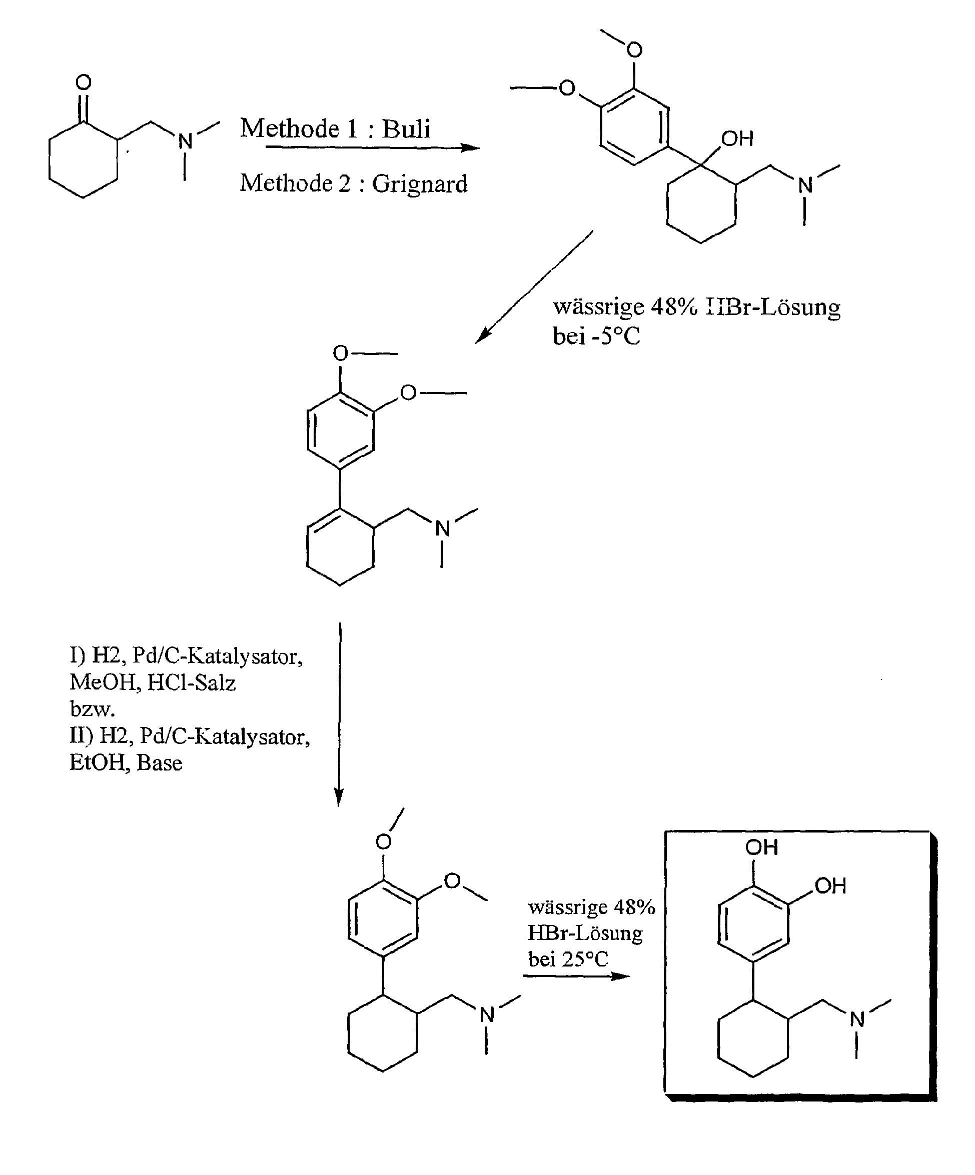

Dabei bezeichnet im übrigen Methode 1 BuLi die dem Fachmann gut bekannte Synthese über BuLi-Reagenzien und Methode 2 Grignard die dem Fachmann gut bekannte Synthese über Mg-Reagenzien.

### Beispiel 8: Herstellung von C-[2-(3-Methoxy-phenyl)-cyclohexyl]-methylamin.

Die Herstellung von C-[2-(3-Methoxy-phenyl)-cyclohexyl]-methylamin erfolgte nach folgenden Reaktionsschema:

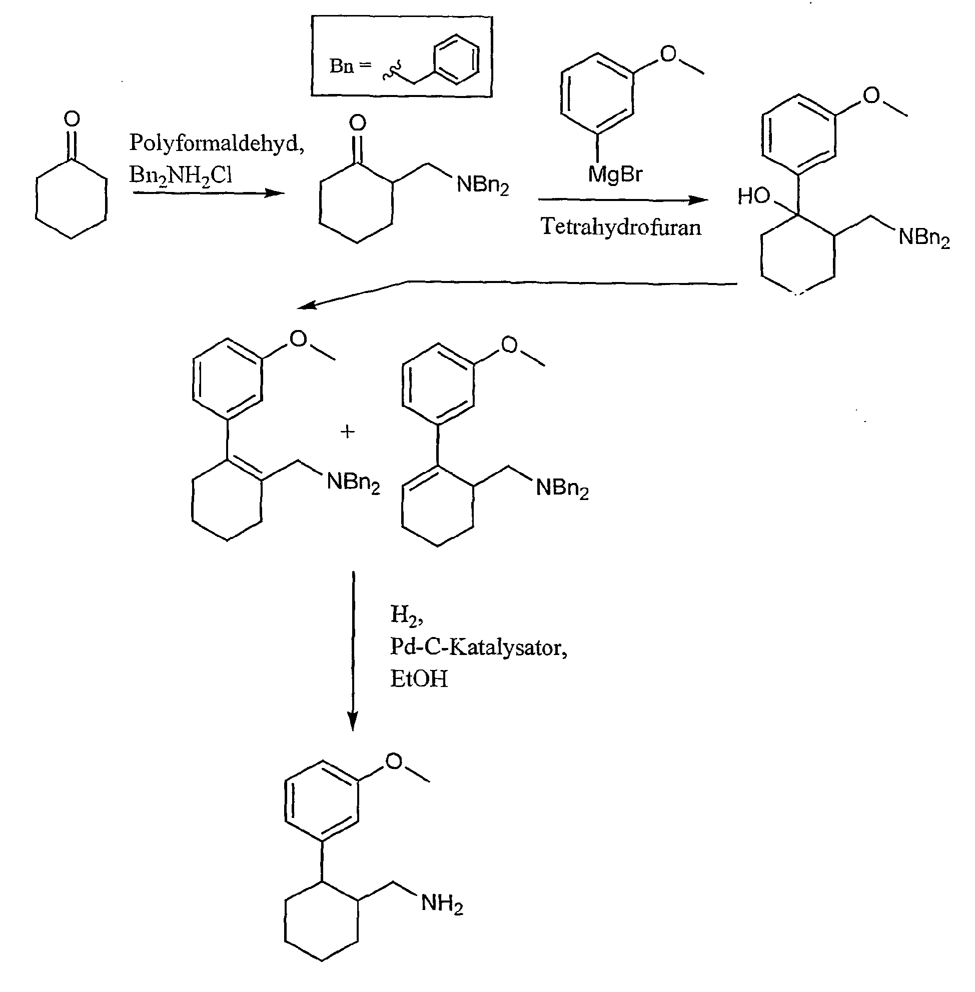

### Beispiel 9: Herstellung von (1R,2R)-3-(2-Aminomethyl-cyclohexyl)-phenol.

Die Herstellung von 3-(2-Aminomethyl-cyclohexyl)-phenol bzw. (1R,2R)-3-(2-Aminomethyl-cyclohexyl)-phenol erfolgte nach folgenden Reaktionsschema:

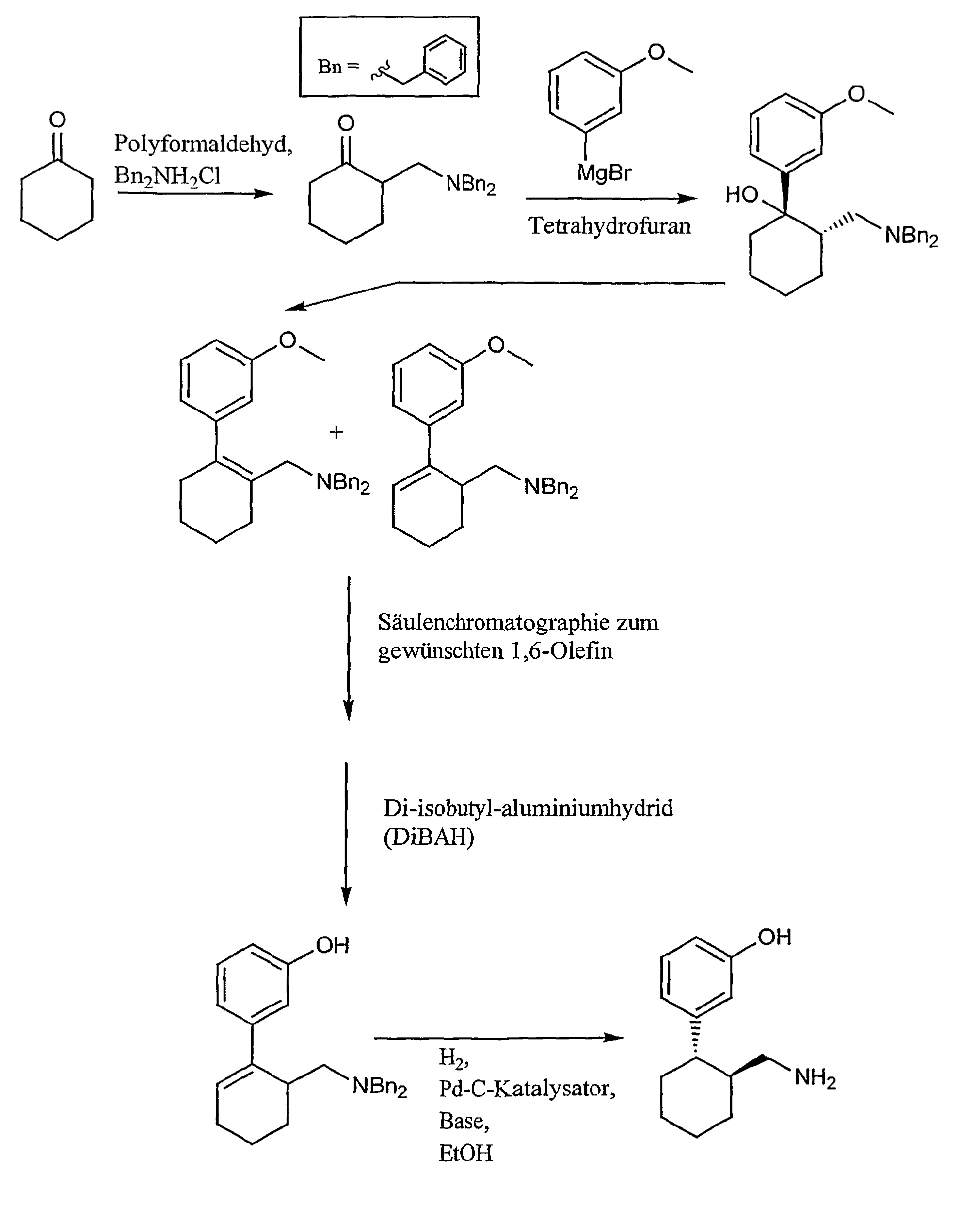

### Beispiel 10 In-Vitro-Isolierung der Metabolite:

Es wurde [2-(3-Methoxyphenyl)-cyclohexylmethyl]-dimethylamin; Hydrochlorid und in einem anderen Beispiel (1R,2R)-3-(2-Dimethylaminomethyl-cychlohexyl)-phenol; Hydrochlorid in TRIS/HCl-Puffer pH 7,4 gelöst. Dann wurden MgCl sowie gegebenenfalls die anderen literaturbekannten notwendigen Cofaktoren für CytochromP450 (CytP450) hinzugefügt und mit CytP450 3A4 (N-Demethylierung) und/oder CytP450 2D6 (O-Demethylierung) bei 37°C inkubiert. Anschließend wurde der Ansatz über HPLC aufgetrennt, die Metabolite in den Fraktionen über NMR identifiziert und dann aus den Fraktionen isoliert.

### Beispiel 11 In-Vivo-Isolierung der Metabolite:

Einem Säugetier wurden [2-(3-Methoxyphenyl)-cyclohexylmethyl]-dimethylamin; Hydrochlorid und in einem weiteren Beispiel (1R,2R)-3-(2-Dimethylaminomethyl-cychlohexyl)-phenol; Hydrochlorid injiziert. Dem Säugetier wurde Blut entnommen, dieses nach Abtrennung korpuskulärer Bestandteile über HPLC aufgetrennt, die Metabolite in den Fraktionen über NMR identifiziert und dann aus den Fraktionen isoliert.

### Beispiel 12 µ-Opioid Rezeptor-Affinität und NA- und 5HT-Uptake Hemmkomponenten:

Es wurden neben der für eine Opiodsubstanz entscheidenden Bindung an den µ-Opioid Rezeptor auch die NA- und 5HT-Uptake Hemmkomponenten auch für zwei klinisch verwendete Antidepressiva, Fluoxetin und Desipramin, untersucht. Die Ergebnisse dieser, in der Fachliteratur in ihrer Durchführung hinlänglich bekannten Standardtests sind in Tabelle 1 zusammengefaßt:

**Tabelle 1: Uptakehemmung und Opioidrezeptorbindung**

| | **(1R,2R)-3-(2-Dimethylaminomethylcyclohexyl)-phenol; Hydrochlorid** | **(1R,2R)-3-(2-Methylaminomet hyl-cyclohexyl)-phenol ; Hydrochlorid** | **Fluoxetin** | **Desipramin** |
|---|---|---|---|---|
| | Kᵢ-Werte (µM) | | | |
| µ-Opioid Rezeptor | 0,038 (Ratte) | 0,91 (Human) | unw. | unw. |
| NA-Uptakehemmung | 0,16 | 0,56 | 0,53 | 0,0011 |
| 5HT-Uptakehemmung | 0,05 | 9,41 | 0,026 | 1,44 |

Die 5HT-Reuptakehemmung gerade von (1R,2R)-3-(2-Dimethylaminomethyl-cyclohexyl)-phenol liegt in der gleichen Größenordnung wie die µ-Opioidrezeptorbindung der Substanz. Damit besitzt die Substanz ein exakt ausbalanciertes Verhältnis von µ-Opioidkomponente und Uptakehemmung und besitzt damit auch das Potential für eine besonders effektive Schmerzhemmung. Die Uptakehemmung ist in der Größenordnung von klinisch verwendete Antidepressiva und führt damit zu einer eigenständigen antidepressiven Wirkkomponente, wobei die Substanz eine relativ starke Hemmwirkung auf den Noradrenalin- und insbesondere Serotonin-Reuptake besitzt.

### Beispiel 13 "Tail Suspension-Test":

Der "Tail Suspension Test" detektiert antidepressive und anxiolytische Aktivität und folgt der Methode von Porsolt et al. (Porsolt R.D., Bertin A., Jalfre M., Behaviourial despair in mice: a primary screening test for antidepressants. Arch. Int. Pharmacodyn. Ther., 229, 327-336, 1977) und Stéru et al. (Stéru L., .Chermat R., Thierry B, Simon P. The Tail Suspension Test: a new method for screening antidepressants in mice. Psychopharmacology, 85, 367-370, 1985). Nagetiere, die man am Schwanz herabhängen läßt, werden schnell immobil. Antidepressiva verringern die Dauer der Immobilität, während Tranquilantien die Dauer der Immobilität verlängern. Das Verhalten der Tiere wurde über 6 Minuten beobachtet, vorzugsweise mit einem computerisierten Gerät (Itemac-TST) entwickelt von Stéru et al. (1985) (s.o.). Mehrere Mäuse wurden parallel untersucht und die Immobilitätsdauer bestimmt. Dieser Parameter ist analog zu dem im "behavioral despair"-Test (Stéru L., Chermat R., Thierry B., Mico J.A., Lenègre A., Stéru M., Simon P. Porsolt R.D., The automated Tail Suspension Test: a computerized device which differentiates psychotropic drugs. Prog. Neuropsychopharmacol. Exp. Psychiatry, 11, 659-671, 1987) verwendeten. 10-20 Mäuse wurden pro Gruppe untersucht. Morphin, (1R,2R)-3-(2-Dimethylaminomethyl-cyclohexyl)-phenol;Hydrochlorid und Desipramin wurden 30 Minuten vor dem Test i.p. gegeben und Naloxon i.v.

Dieses Verhaltensmodell ist in der Lage, die antidepressive Wirkung der Uptakehemmkomponente bei gleichzeitiger Anwesenheit einer Opioidwirkung zu zeigen. In diesem Verhaltensmodell führen Antidepressiva wie auch in anderen Verhaltensmodellen zu einer Verkürzung der Immobilitätsphase, während Opioide wie Morphin die Immobilitätsphase verlängern. Beim Vorhandensein beider Wirkkomponenten überlagern sich beide Effekte, so daß nur noch eine geringe Motilitätsveränderung resultiert. Das wurde dadurch aufgehoben, daß die Opiatkomponente durch Vorbehandlung mit Naloxon weggeblockt wurde. Nach der Naloxonvorbehandlung trat die stimulierende Wirkung von (1R,2R)-3-(2-Dimethylaminomethyl-cyclohexyl)-phenol, die ohne Vorbehandlung nur gering war, deutlich in Erscheinung. Darum ist die Substanz, insbesondere in Kombination mit einer Schmerztherapie sehr geeignet. Die Ergebnisse dieser Untersuchungen zusammen mit Referenzsubstanzen sind in der Tabelle 2 zusammengefaßt.

**Tabelle 2: Wirkung von (1R,2R)-3-(2-Dimethylaminomethyl-cyclohexyl)-phenol, Imipramin und Morphin in "Tail Suspension" Test an der Maus (10 - 20 Tiere/Gruppe)**

| **Substanz** | **Dosis** | **Imobilitätsdauer** |
|---|---|---|
| | (mg/kg i.p.) | |
| | | % Änderung gegenüber der Lösungsmittelkontrolle |
| (1R,2R)-3-(2-Dimethylaminomethyl-cyclohexyl)-phenol;Hydrochlorid | 4,64 | +6% |
| | 10,0 | +12 % |
| | 21,5 | +9 % |
| | 31,6 | -13 % |
| (1R,2R)-3-(2-Dimethylaminomethyl-cyclohexyl)-phenol;Hydrochlorid | 21,5 | -90 % |
| | 31,6 | -85 % |
| + 1,0 mg/kg i.v. Naloxon | | |
| Naloxon 1,0 mg/kg i.v. | - | -15 % |
| Morphin | 4,64 | +90% |
| | 10,0 | +145 % |
| | 21,5 | +169 % |
| Imipramin | 31,6 | -61 % |

Die Tab. 2 zeigt, daß (1R,2R)-3-(2-Dimethylaminomethyl-cyclohexyl)-phenol;Hydrochlorid erst nach Vorbehandlung mit Naloxon eine starke Abnahme der Immobilitätsdauer bewirkt, wie sie auch bei Imipramin gefunden wurde. Opioide führen, wie für Morphin gezeigt, zu einer Verlängerung der Immobilitätsdauer. Dies erklärt warum (1R,2R)-3-(2-Dimethylaminomethyl-cyclohexyl)-phenol ohne Naloxon-Vorbehandlung als Nettoeffekt von Uptakehemmung und Opioidwirkung nur eine geringe Änderung der Immobilitätsdauer im Vergleich zur Lösungsmittelkontrolle hervorruft.

Nach den vorliegenden Befunden ist überraschenderweise (1R,2R)-3-(2-Dimethylaminomethyl-cyclohexyl)-phenol das erste Opiodanalgetikum, das eine relevante und offensichtlich auch klinisch nutzbare antidepressive Wirkkomponente besitzt.

### Beispiel 14: Parenterale Applikationsform

1 g (1R,2R)-3-(2-Dimethylaminomethyl-cyclohexyl)-phenol ; Hydrochlorid wird in 1 I Wasser für Injektionszwecke bei Raumtemperatur gelöst und anschließend durch Zugabe von NaCl auf isotone Bedingungen eingestellt.

## Patentansprüche

1. Verwendung von
■ 3-(2-Dimethylaminomethyl-cyclohexyl)-phenol,
■ (1R,2R)- 3-(2-Dimethylaminomethyl-cyclohexyl)-phenol,
■ [2-(3-Methoxyphenyl)-cyclohexylmethyl]-dimethylamin,
■ (1R, 2R)-[2-(3-Methoxyphenyl)-cyclohexylmethyl]-dimethylamin,
■ Schwefelsäure mono-[3-(2-dimethylaminomethyl-cyclohexyl)-phenyl]ester,
■ Schwefelsäure mono-(1R,2R)-[3-(2-dimethylaminomethyl-cyclohexyl)-phenyl]ester,
■ 3-(2-Methylaminomethyl-cyclohexyl)-phenol,
■ (1R,2R)-3-(2-Methylaminomethyl-cyclohexyl)-phenol,
■ 3-(2-Dimethylaminomethyl-cyclohexyl)-phenol-N-oxid,
■ (1R,2R)-3-(2-Dimethylaminomethyl-cyclohexyl)-phenol-N-oxid,
■ 6-[3-(2-Dimethylaminomethyl-cyclohexyl)-phenoxy]-3,4,5-trihydroxy-tetrahydropyran-2-carbonsäure,
■ 6-[(1R,2R)-3-(2-Dimethylaminomethyl-cyclohexyl)-phenoxy]-3,4,5-trihydroxy-tetrahydropyran-2-carbonsäure,
■ 4-(2-Dimethylaminomethyl-cyclohexyl)-catechol,
■ (1R,2R)-4-(2-Dimethylaminomethyl-cyclohexyl)-catechol,
■ 3-(2-Aminomethyl-cyclohexyl)-phenol,
■ (1R,2R)-3-(2-Aminomethyl-cyclohexyl)-phenol,
■ 4-(2-Dimethylaminomethyl-cyclohexyl)-benzene-1,2-diol,
■ (1R,2R)- 4-(2-Dimethylaminomethyl-cyclohexyl)-benzene-1,2-diol,
■ C-[2-(3-Methoxy-phenyl)-cyclohexyl]-methylamin,
■ (1R,2R)-C-[2-(3-Methoxy-phenyl)-cyclohexyl]-methylamin,
■ [2-(3-Methoxy-phenyl)-cyclohexylmethyl]-methyl-amin,
■ (1R,2R)-[2-(3-Methoxy-phenyl)-cyclohexylmethyl]-methyl-amin,
■ [2-(3-Methoxy-phenyl)-cyclohexylmethyl]-dimethyl-amin; N-oxid oder
■ (1R,2R)-[2-(3-Methoxy-phenyl)-cyclohexylmethyl]-dimethyl-amin; N-oxid;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
zur Herstellung eines Arzneimittels zur Behandlung von Depressionen.

2. Verwendung gemäß Anspruch 1, **dadurch gekennzeichnet, daß** die verwendeten Verbindungen als R, R, vorzugsweise 1R, 2R Stereoisomere vorliegen.

3. Metabolite von 3-(2-Dimethylaminomethyl-cyclohexyl)-phenol ausgewählt aus
■ Schwefelsäure mono-[3-(2-dimethylaminomethyl-cyclohexyl)-phenyl]ester,
■ Schwefelsäure mono-(1R,2R)-[3-(2-dimethylaminomethyl-cyclohexyl)-phenyl]ester,
■ 3-(2-Methylaminomethyl-cyclohexyl)-phenol,
■ (1R,2R)-3-(2-Methylaminomethyl-cyclohexyl)-phenol,
■ 3-(2-Dimethylaminomethyl-cyclohexyl)-phenol-N-oxid,
■ (1R,2R)-3-(2-Dimethylaminomethyl-cyclohexyl)-phenol-N-oxid,
■ 6-[3-(2-Dimethylaminomethyl-cyclohexyl)-phenoxy]-3,4,5-trihydroxy-tetrahydropyran-2-carbonsäure,
■ 6-[(1R,2R)-3-(2-Dimethylaminomethyl-cyclohexyl)-phenoxy]-3,4,5-trihydroxy-tetrahydropyran-2-carbonsäure,
■ 4-(2-Dimethylaminomethyl-cyclohexyl)-catechol,
■ (1R,2R)-4-(2-Dimethylaminomethyl-cyclohexyl)-catechol,
■ 3-(2-Aminomethyl-cyclohexyl)-phenol,
■ (1R,2R)-3-(2-Aminomethyl-cyclohexyl)-phenol,
■ 4-(2-Dimethylaminomethyl-cyclohexyl)-benzene-1,2-diol,
■ (1R,2R)- 4-(2-Dimethylaminomethyl-cyclohexyl)-benzene-1,2-diol,
■ C-[2-(3-Methoxy-phenyl)-cyclohexyl]-methylamin,
■ (1R,2R)-C-[2-(3-Methoxy-phenyl)-cyclohexyl]-methylamin,
■ [2-(3-Methoxy-phenyl)-cyclohexylmethyl]-methyl-amin,
■ (1R,2R)-[2-(3-Methoxy-phenyl)-cyclohexylmethyl]-methyl-amin,
■ [2-(3-Methoxy-phenyl)-cyclohexylmethyl]-dimethyl-amin; N-oxid oder
■ (1R,2R)-[2-(3-Methoxy-phenyl)-cyclohexylmethyl]-dimethyl-amin; N-oxid;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

4. Metabolite gemäß Anspruch 3, **dadurch gekennzeichnet, daß** die Verbindungen als R, R, vorzugsweise 1R, 2R Stereoisomere vorliegen.

5. Arzneimittel enthaltend als Wirkstoff wenigstens eine Verbindung gemäß einem der Ansprüche 3 oder 4 sowie gegebenenfalls Zusatz-und/oder Hilfsstoffe.

6. Verwendung einer Verbindung gemäß einem der Ansprüche 3 oder 4 zur Herstellung eines Arzneimittels zur Behandlung von Schmerz, insbesondere akutem, viszeralem, chronischem oder neuropathischem Schmerz oder Krebsschmerz.

7. Verwendung einer Verbindung gemäß einem der Ansprüche 3 oder 4 zur Herstellung eines Arzneimittels zur Behandlung von vermehrtem Harndrang bzw. Harninkontinenz.

## Claims

1. Use of
■ 3-(2-dimethylaminomethylcyclohexyl)-phenol,
■ (1R,2R)-3-(2-dimethylaminomethylcyclohexyl)-phenol,
■ [2-(3-methoxyphenyl)-cyclohexylmethyl]-dimethylamine,
■ (1R, 2R)-[2-(3-methoxyphenyl)-cyclohexylmethyl]-dimethylamine,
■ sulfuric acid mono-[3-(2-dimethylaminomethylcyclohexyl)-phenyl] ester,
■ sulfuric acid mono-(1R,2R)-[3-(2-dimethylaminomethylcyclohexyl)-phenyl] ester,
■ 3-(2-methylaminomethylcyclohexyl)-phenol,
■ (1R,2R)-3-(2-methylaminomethylcyclohexyl)-phenol,
■ 3-(2-dimethylaminomethylcyclohexyl)-phenol N-oxide,
■ (1R,2R)-3-(2-dimethylaminomethylcyclohexyl)-phenol N-oxide,
■ 6-[3-(2-dimethylaminomethylcyclohexyl)-phenoxy]-3,4,5-trihydroxytetrahydropyran-2-carboxylic acid,
■ 6-[(1R,2R)-3-(2-dimethylaminomethylcyclohexyl)-phenoxy]-3,4,5-trihydroxytetrahydropyran-2-carboxylic acid,
■ 4-(2-dimethylaminomethylcyclohexyl)-catechol,
■ (1R,2R)-4-(2-dimethylaminomethylcyclohexyl)-catechol,
■ 3-(2-aminomethylcyclohexyl)-phenol,
■ (1R,2R)-3-(2-aminomethylcyclohexyl)-phenol,
■ 4-(2-dimethylaminomethylcyclohexyl)-benzene-1,2-diol,
■ (1R,2R)- 4-(2-dimethylaminomethylcyclohexyl)-benzene-1,2-diol,
■ C-[2-(3-methoxyphenyl)-cyclohexyl]-methylamine,
■ (1R,2R)-C-[2-(3-methoxyphenyl)-cyclohexyl]-methylamine,
■ [2-(3-methoxyphenyl)-cyclohexylmethyl]-methylamine,
■ (1R,2R)-[2-(3-methoxyphenyl)-cyclohexylmethyl]-methylamine,
■ [2-(3-methoxyphenyl)-cyclohexylmethyl]-dimethylamine N-oxide or
■ (1R, ,2R)-[2-(3-methoxyphenyl)-cyclohexylmethyl]-dimethylamine N-oxide;
optionally in the form of the racemates thereof, the pure stereoisomers thereof, in particular enantiomers or diastereomers, or in the form of mixtures of the stereoisomers, in particular of the enantiomers or diastereomers, in any desired mixing ratio;
in the form as prepared or in the form of the acids or bases thereof or in the form of the salts thereof, in particular the physiologically acceptable salts, or in the form of the solvates thereof, in particular the hydrates;
for producing a medicament for treating depression.

2. Use according to claim 1, **characterised in that** the compounds used are present as R,R, preferably 1R,2R, stereoisomers.

3. Metabolites of 3-(2-dimethylaminomethylcyclohexyl)-phenol selected from
■ sulfuric acid mono-[3-(2-dimethylaminomethylcyclohexyl)-phenyl] ester,
■ sulfuric acid mono-(1R,2R)-[3-(2-dimethylaminomethylcyclohexyl)-phenyl] ester,
■ 3-(2-methylaminomethylcyclohexyl)-phenol,
■ (1R,2R)-3-(2-methylaminomethylcyclohexyl)-phenol,
■ 3-(2-dimethylaminomethylcyclohexyl)-phenol N-oxide,
■ (1R,2R)-3-(2-dimethylaminomethylcyclohexyl)-phenol N-oxide,
■ 6-[3-(2-dimethylaminomethylcyclohexyl)-phenoxy]-3,4,5-trihydroxytetrahydropyran-2-carboxylic acid,
■ 6-[(1R,2R)-3-(2-dimethylaminomethylcyclohexyl)-phenoxy]-3,4,5-trihydroxytetrahydropyran-2-carboxylic acid,
■ 4-(2-dimethylaminomethylcyclohexyl)-catechol,
■ (1R,2R)-4-(2-dimethylaminomethylcyclohexyl)-catechol,
■ 3-(2-aminomethylcyclohexyl)-phenol,
■ (1R,2R)-3-(2-aminomethylcyclohexyl)-phenol,
■ 4-(2-dimethylaminomethylcyclohexyl)-benzene-1,2-diol,
■ (1R,2R)- 4-(2-dimethylaminomethylcyclohexyl)-benzene-1,2-diol,
■ C-[2-(3-methoxyphenyl)-cyclohexyl]-methylamine,
■ (1R,2R)-C-[2-(3-methoxyphenyl)-cyclohexyl]-methylamine,
■ [2-(3-methoxyphenyl)-cyclohexylmethyl]-methylamine,
■ (1R,2R)-[2-(3-methoxyphenyl)-cyclohexylmethyl]-methylamine,
■ [2-(3-methoxyphenyl)-cyclohexylmethyl]-dimethylamine N-oxide
or
■ (1R, ,2R)-[2-(3-methoxyphenyl)-cyclohexylmethyl]-dimethylamine N-oxide;
optionally in the form of the racemates thereof, the pure stereoisomers thereof, in particular enantiomers or diastereomers, or in the form of mixtures of the stereoisomers, in particular of the enantiomers or diastereomers, in any desired mixing ratio; in the form as prepared or in the form of the acids or bases thereof or in the form of the salts thereof, in particular the physiologically acceptable salts, or in the form of the solvates thereof, in particular the hydrates.

4. Metabolites according to claim 3, **characterised in that** the compounds used are present as R,R, preferably 1R,2R, stereoisomers.

5. A medicament containing as active ingredient at least one compound according to either one of claim 3 or claim 4 and optionally additives and/or auxiliary substances.

6. Use of a compound according to either one of claim 3 or claim 4 for producing a medicament for treating pain, in particular acute, visceral, chronic or neuropathic pain or cancer pain.

7. Use of a compound according to either one of claim 3 or claim 4 for producing a medicament for treating increased urinary urgency or urinary incontinence.

## Revendications

1. Utilisation de
- 3-(2-diméthylaminométhylcyclohexyl)-phénol,
- (1R,2R)-3-(2-diméthylaminométhylcyclohexyl)-phénol,
- [2-(3-méthoxyphényl)-cyclohexylméthyl]-diméthylamine,
- (1R,2R)-[2-(3-méthoxyphényl)-cyclohexylméthyl]-diméthylamine,
- ester mono-[3-(2-diméthylaminométhylcyclohexyl)-phénylique] de l'acide sulfurique,
- ester mono-(1R,2R)-[3-(2-diméthylaminométhylcyclohexyl)-phénylique] de l'acide sulfurique,
- 3-(2-méthylaminométhylcyclohexyl)-phénol,
- (1R,2R)-3-(2-méthylaminométhylcyclohexyl)-phénol,
- N-oxyde de 3-(2-diméthylaminométhylcyclohexyl)-phénol
- N-oxyde de (1R,2R)-3-(2-diméthylaminométhylcyclohexyl)-phénol,
- acide 6-[3-(2-diméthylaminométhylcyclohexyl)-phénoxy]-3,4,5-trihydroxytétrahydropyranne-2-carboxylique,
- acide 6-[(1R,2R)-3-(2-diméthylaminométhylcyclohexyl)-phénoxy]-3,4,5-trihydroxytétrahydropyranne-2-carboxylique,
- 4-(2-diméthylaminométhylcyclohexyl)-catéchol,
- (1R,2R)-4-(2-diméthylaminométhylcyclohexyl)-catéchol,
- 3-(2-aminométhylcyclohexyl)-phénol,
- (1R,2R)-3-(2-aminométhylcyclohexyl)-phénol,
- 4-(2-diméthylaminométhylcyclohexyl)-benzène-1,2-diol,
- (1R,2R)-4-(2-diméthylaminométhylcyclohexyl)-benzène-1,2-diol,
- C-[2-(3-méthoxyphényl)-cyclohexyl]-méthylamine,
- (1R,2R)-C-[2-(3-méthoxyphényl)-cyclohexyl]-méthylamine,
- [2-(3-méthoxyphényl)-cyclohexylméthyl]-méthylamine,
- (1R,2R)-[2-(3-méthoxyphényl)-cyclohexylméthyl]-méthylamine,
- [2-(3-méthoxyphényl)-cyclohexylméthyl]-diméthylamine ; N-oxyde ou
- (1R,2R)-[2-(3-méthoxyphényl)-cyclohexylméthyl]-diméthylamine ; N-oxyde ;
le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ; sous la forme préparée ou sous forme de leurs acides ou de leurs bases ou sous forme de leurs sels, en particulier des sels physiologiquement acceptables, ou sous forme de leurs solvates, en particulier des hydrates ;
pour la préparation d'un médicament destiné au traitement de dépressions.

2. Utilisation selon la revendication 1, **caractérisée en ce que** les composés utilisés se trouvent sous forme de stéréo-isomères R,R, de préférence de stéréo-isomères 1R,2R.

3. Métabolites de 3-(2-diméthylaminométhylcyclohexyl)-phénol choisis parmi
- l'ester mono-[3-(2-diméthylaminométhylcyclohexyl)-phénylique] de l'acide sulfurique,
- l'ester mono-(1R,2R)-[3-(2-diméthylaminométhylcyclohexyl)-phénylique] de l'acide sulfurique,
- le 3-(2-méthylaminométhylcyclohexyl)-phénol,
- le (1R,2R)-3-(2-méthylaminométhylcyclohexyl)-phénol,
- le N-oxyde de 3-(2-diméthylaminométhylcyclohexyl)-phénol,
- le N-oxyde de (1R,2R)-3-(2-diméthylaminométhylcyclohexyl)-phénol,
- l'acide 6-[3-(2-diméthylaminométhylcyclohexyl)-phénoxy]-3,4,5-trihydroxytétrahydropyranne-2-carboxylique,
- l'acide 6-[(1R,2R)-3-(2-diméthylaminométhylcyclohexyl)-phénoxy]-3,4,5-trihydroxytétrahydropyranne-2-carboxylique,
- le 4-(2-diméthylaminométhylcyclohexyl)-catéchol,
- le (1R,2R)-4-(2-diméthylaminométhylcyclohexyl)-catéchol,
- le 3-(2-aminométhylcyclohexyl)-phénol,
- le (1R,2R)-3-(2-aminométhylcyclohexyl)-phénol,
- le 4-(2-diméthylaminométhylcyclohexyl)-benzène-1,2-diol,
- le (1R,2R)-4-(2-diméthylaminométhylcyclohexyl)-benzène-1,2-diol,
- la C-[2-(3-méthoxyphényl)-cyclohexyl]-méthylamine,
- la (1R,2R)-C-[2-(3-méthoxyphényl)-cyclohexyl]-méthylamine,
- la [2-(3-méthoxyphényl)-cyclohexylméthyl]-méthylamine,
- la (1R,2R)-[2-(3-méthoxyphényl)-cyclohexylméthyl]-méthylamine,
- le [2-(3-méthoxyphényl)-cyclohexylméthyl]-diméthylamine ; N-oxyde ou
- le (1R,2R)-[2-(3-méthoxyphényl)-cyclohexylméthyl]-diméthylamine ; N-oxyde ;
le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ; sous la forme préparée ou sous forme de leurs acides ou de leurs bases ou sous forme de leurs sels, en particulier des sels physiologiquement acceptables, ou sous forme de leurs solvates, en particulier des hydrates.

4. Métabolites selon la revendication 3, **caractérisés en ce que** les composés se trouvent sous forme de stéréo-isomères R,R, de préférence de stéréo-isomères 1R,2R.

5. Médicament contenant comme substance active au moins un composé selon l'une quelconque des revendications 3 ou 4 ainsi que le cas échéant des additifs et/ou des adjuvants.

6. Utilisation d'un composé selon l'une quelconque des revendications 3 ou 4 pour la préparation d'un médicament destiné au traitement de la douleur, en particulier de la douleur aiguë, viscérale, chronique ou neuropathique ou de la douleur cancéreuse.

7. Utilisation d'un composé selon l'une quelconque des revendications 3 ou 4 pour la préparation d'un médicament destiné au traitement d'un besoin accru d'uriner ou de l'incontinence urinaire.
